# EUROPEAN PATENT SPECIFICATION

(11) **EP 0 235 957 B1**
(45) Date of publication and mention of the grant of the patent: **07.04.1993**
(21) Application number: 87300962.5
(22) Date of filing: 03.02.1987
(51) Int. Cl.: A61K 31/20

(54) **Botulinus toxin neutralizer**
Neutralisierung von Botulinus-Toxin
Neutralisant de la toxine Botulinus

(30) Priority: 03.02.1986 JP 20066/86
(43) Date of publication of application: 09.09.1987
(73) Proprietor: KABUSHIKI KAISHA YAKULT HONSHA, Minato-ku Tokyo 105 (JP)
(72) Inventor: Nagai, Yoshitaka, Setagaya-ku Tokyo (JP); Takamizawa, Koutaro, Irima Saitama (JP); Tanaka, Ryuichiro, Tachikawa Tokyo (JP); Takayama, Hiroo, Tokorozawa Saitama (JP); Sakurai, Toshizo, Tokyo (JP); Mutai, Masahiko, Tokyo (JP)
(74) Representative: Brewer, Leonard Stuart

(56) References cited:
- JOURNAL OF NEUROCHEMISTRY, vol. 18, 1971, GB; L.L. SIMPSON et al., pp. 1751-1759#
- FEBS, vol. 201, no. 2, June 1986, Federation of European Biochemical Societies; K. TAKAMIZAWA et al., pp. 229-232#
- JOURNAL OF FOOD PROTECTION, vol. 45, no. 12, October 1982; M. DYMICKY et al., pp. 1117-1119#

## Description

The present invention relates to the manufacture of a botulinum toxin neutralizer which is effective for the prevention and treatment of botulinum intoxication.

Botulinum toxin is a proteinous exotoxin, secreted by Clostridium botulinum which is widely distributed in soil, and acts at the tips of the motor nerve endings at the neuromuscular junctions. When orally ingested into the human body, the botulinum toxin gives rise to intoxication called botulism, which is accompanied by paralytic symptoms. Botulism breaks out when the neurotoxin is absorbed from the alimentary tract and combines selectively with the presynaptic membranes at the peripheries of the neuromuscular junctions. The toxin thus interferes with the release of acetylcholine from the chlorinergic motor nerve endings, and eventually with conduction of nerve impulses in the terminal branches of the motor nerves to cause the neuromuscular relaxation and paralysis which is characteristic of botulism. In the worst cases, death may occur from paralysis of the respiratory muscles.

As to the pathogenic mechanism of the botulinum toxin, it is established that the ganglioside GT1b, an acidic glycolipid present in the presynaptic membrane acts as a receptor for the toxin. While other types of gangliosides such as the gangliosides GQ1b, GD1b and GD1a also have the ability to combine with botulinum toxin, these gangliosides are less potent than the ganglioside GT1b in combining with botulinum toxin, and for this reason are considered less responsible for botulism.

The treatment of botulism is extremely difficult, and at the present time there is practically no other method of treatment than to combat the symptoms once botulism has broken out. In view of the pathogenic mechanism of the botulinum toxin as discussed above, the first conceivable approach to the treatment of botulism may be to use the ganglioside GTb1 as an antagonistic receptor for the botulinum toxin, since the substance is capable of combining directly with the toxin. For this purpose, the ganglioside GTb1 may be orally administered to the human body for direct attachment to the botulinum toxin so as to prevent the onset of the toxic effects thereof. There is however a problem with this approach, in that the only presently available source of the ganglioside GTb1 is the bovine brain, which is so expensive that the method of treating botulism using such a ganglioside has seldom been put into practice.

Against this background, it is an object of the present invention to provide an economical botulinum toxin neutralizer which acts as if it were an antagonistic toxin receptor for the treatment of botulism, and which will thus facilitate the prevention and treatment of botulism.

The present invention has been evolved as the result of extensive researches conducted in quest of substances with the ability to neutralize the botulinum toxin, and has emerged from the discovery that there is a family of fatty acids which have a potent capacity to combine with and neutralize the botulinum toxin.

According to the invention, there is provided the use of fatty acids having at least 12 carbon atoms for the manufacture of a medicament or of a medicated food additive or food for the treatment of botulism.

The fatty acid (s) used may include unsaturated fatty acids such as for example oleic acid with 18 carbon atoms (C18:1), CH₃(CH₂)₇CH=CH(CH₂)₇COOH. It is however preferred to use a fatty acid which possessed one or more of the features:
(A) it is a saturated fatty acid;
(B) it is a straight-chain fatty acid; and
(C) it has 16-22 carbon atoms.

Examples of such fatty acids include straight-chain saturated fatty acids such as, typically:
- lauric acid with 12 carbon atoms (C12:0), CH₃(CH₂)₁₀COOH;
- myristic acid with 14 carbon atoms (C14:0), CH₃(CH₂)₁₂COOH;
- palmitic acid with 16 carbon atoms (C16:0), CH₃(CH₂)₁₄COOH;
- stearic acid with 18 carbon atoms (C18:0), CH₃(CH₂)₁₆COOH;
- nonadecanoic acid with 19 carbon atoms (C19:0), CH₃(CH₂)₁₇COOH;
- arachidic acid with 20 carbon atoms (C20:0), CH₃(CH₂)₁₈COOH; and
- behenic acid with 22 carbon atoms (C22:0), CH₃(CH₂)₂₀COOH.

Preferred examples of fatty acids having from 16 to 22 carbon atoms inclusive to be used as a botulinum toxin neutralizer thus include saturated fatty acids such as palmitic acid, stearic acid, nonadecanoic acid, arachidic acid, a fatty acid with 21 carbon atoms (C21:0), and behenic acid, as well as unsaturated fatty acids such as oleic acid (C18:1).

The most preferred of these are palmitic acid and stearic acid, because they possess particularly excellent botulinum toxin neutralizing capacity and they are widely available on a commercial basis, as they occur abundantly in various animal and vegetable tissues.

While a botulinum toxin neutralizer manufactured according to the present invention need contain only one of the fatty acids having at least 12 and preferably 16 to 22 carbon atoms as above noted, if desired two or more such fatty acids may be used in combination.

The fatty acid which is used as a botulinum toxin neutralizer according to the present invention acts as if it were an antagonistic receptor for botulinum toxin, and when it encounters botulinum toxin in vivo the fatty acid combines directly with the toxin and disables it from combining with the neuromuscular tissues of, for example, the human body so as thus to prevent the outbreak of botulism. The toxin thus neutralized and affixed to the fatty acid is, as it is, excreted from the human body.

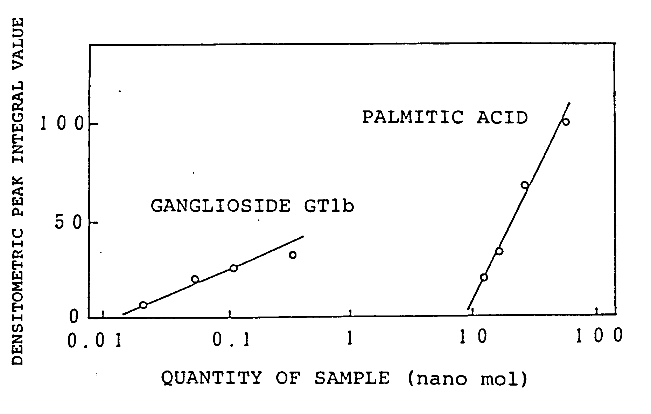

Other important aspects of the present invention will be understood from the following description read in conjunction with the accompanying drawing. The drawing shows the results of tests conducted with palmitic acid and ganglioside GT1b, using the TLC (thin layer chromatography) immunostaining method to evaluate their abilities to combine with the botulinum type A toxin, so as thus to make a comparison between the neutralizing ability of a botulinum toxin neutralizer according to the present invention and that of a known botulinum toxin neutralizing substance.

The performance of these higher fatty acids for neutralizing the botulinum toxin have been confirmed by enzyme-immunostaining and densitometric determination techniques, using the known thin layer chromotography (TLC) immunostaining method on thin layer chromatogram plates (H. Higashi et al, J. Biochem. 95, 1517-1520, 1984). A description will hereinafter be given regarding the TLC immunostaining procedures thus carried out upon a variety of fatty acids, including those which can be used singly or in combination as a botulinum toxin neutralizer according to the present invention.

Prior to these TLC immunostaining procedures, sample solutions respectively containing various fatty acids including those having less than 12 carbon atoms and those having 12 or more carbon atoms were prepared. Each of these fatty-acid containing sample solutions was dissolved in a solution of chloroform and methanol mixed in the ratio of 2:1 on a volume basis. The resultant mixture was spotted onto a plastic thin-layer-chromatogram plate (Polygram SilG, manufactured by Marchery-Nagel, West Germany; Mori et al, Biochem. Biophys. Res. Commun. 108, 926-932, 1982), which was then developed with a solution of n-hexane, ether and acetic acid mixed in the ratios of 80:30:1 on a volume basis, and was thereafter dried in air. The plate was subsequently dipped in phosphate-buffered saline containing 1% egg albumin, 1% polyvinylpyrrolidone and 0.02% sodium azide (NaN₃) and was therein incubated at 37°C for an hour. After the incubation, the plate was washed several times with phosphate-buffered saline containing 3% polyvinylpyrrolidone. An aqueous solution containing the botulinum type A toxin in the concentration of 0.5 microgram/milliliter was applied to the resultant plate in a quantity of 100 microliter/cm², whereupon the plate was allowed to stand overnight at 4°C to enable the reaction to proceed. The plate was then washed with phosphate-buffered saline containing 3% polyvinylpyrrolidone and was then incubated at 37°C for two hours with a rabbit antitoxin antiserum applied to the plate. After the incubation, the plate was washed sufficiently with phosphate-buffered saline containing 0.1% Tween 20 (commercially available from Atlas Powder Company) and was then allowed to stand for incubation at 37°C for 15 minutes in phosphate-buffered saline containing 1% egg albumin, 1% polyvinylpyrrolidone and 0.02% sodium aside. The resultant plate was washed with phosphate-buffered saline and was then shaken at 37°C for 2 hours for reaction in a solution of horseradish-peroxidase (HRP) conjugated anti-rabbit immunoglobulin G (IgG) antibody diluted 1000-fold with phosphate-buffered saline containing 3% polyvinylpyrrolidone. After the reaction, the plate was washed in phosphate-buffered salt containing 0.1% Tween 20. Fifty mM Tris-chloride buffer solution (pH 7.4) containing 0.01% hydrogen peroxide, 0.05% 4-chloro-1-naphthol and 200 mM sodium chloride was prepared by the method taught by R. Hawkes et al, (Anal. Biochem. 119, 142-147, 1982). The buffer solution thus prepared was applied as a substrate to the plate, which was thereafter allowed to stand for 15 minutes at room temperature so that coloring reaction proceeds on the plate. After the coloring reaction, the plate was washed with water and was thereupon dried in air to bring the TLC immunostaining procedure to an end.

In this TLC immunostaining procedure, the fatty acid of the sample solution fixed on the chromatogram plate used combines with the botulinum toxin added to the plate if the fatty acid is of the type exhibiting an ability to neutralize the botulinum toxin. The fatty acid thus combined as a receptor with the botulinum toxin further combines with the rabbit antitoxin antiserum through reaction therewith. When the resultant substance then further reacts with the HRP conjugated anti-rabbit IgG antibody, the antibody combines with the anti-rabbit IgG antibody or, in effect, the botulinum toxin contained therein. The horseradish peroxidase thus ultimately combined with the botulinum toxin then reacts with the substrate solution containing hydrogen peroxide and 4-chloro-1-naphthol with the result that there occurs decomposition of the substrate or, more precisely, the 4-chloro-1-naphthol component of the substrate solution. The decomposition of the 4-chloro-1-naphthol results in formation of bluish-purple colored spots on the plate. In the case of the sample solution devoid of botulinum toxin neutralizing ability, the botulinum toxin applied to the chromatogram plate carrying the developed sample solution could not be fixed on the plate and is washed away, together with the rabbit antitoxin antiserum, before the HRP conjugated anti-rabbit IgG antibody is added to the plate. Accordingly, the HRP conjugated anti-rabbit IgG antibody thereafter added to the plate could not stay there, and is therefore also washed away from the plate before the substrate solution is added thereto. Thus, the substrate solution added to the plate used is allowed to remain chemically intact, and will not produce colored spots on the plate.

The areas and concentrations of the bluish-purple colored spots thus produced on the individual chromatogram plates processed with the botulinum toxin-neutralizing fatty acids were determined by densitometric tests using a dual-wave chromatographic scanner (Shimadzu CS-910, manufactured by Shimadzu Seisakusho, Japan) at a wavelength of 570 nanometers. The details of such densitometric determination techniques are described by S. Ando et al, Anal. Biochem. 89, 437-450, 1978. The following table shows the results of the densitometric tests conducted with the sample solutions of various straight-chain saturated fatty acids, wherein the integral values of the peaks observed by the densitometric tests are expressed in terms of index numbers given with the value for the fatty acid (palmitic acid) with 16 carbon atoms used as the base (=100).

| Fatty Acid with Number of Carbon Atoms of: | Integral Value of Densitometric Peaks |
|---|---|
| 10 | 5 |
| 12 | 39 |
| 14 | 67 |
| 16 | 100 |
| 18 | 128 |
| 19 | 100 |
| 20 | 89 |
| 21 | 74 |
| 22 | 72 |
| 23 | 68 |
| 24 | 48 |
| 26 | 49 |

From the above table it will be seen that the botulinum toxin neutralizing abilities of fatty acids vary from one acid to another, depending upon the number of the carbon atoms contained in the acids. Thus, it is apparent from the table that fatty acids having 12 or more carbon atoms are particularly efficacious as botulinum toxin neutralizers, having toxin neutralizing abilities represented by index numbers of more than 30. As noted previously, preferred examples of such fatty acids include saturated fatty acids such as lauric acid with 12 carbon atoms, myristic acid with 14 carbon atoms, palmitic acid with 16 carbon atoms, stearic acid with 18 carbon atoms, nonadecanoic acid with 19 carbon atoms, arachidic acid with 20 carbon atoms and behenic acid with 22 carbon atoms, and unsaturated fatty acids such as oleic acid with 18 carbon atoms (C18:1).

The most preferred of these various fatty acids are those having a number of carbon atoms within the range of from 16 to 22, in view of their toxin neutralizing abilities represented by the prominently high index numbers indicated in the above table. Such fatty acids include saturated fatty acids such as palmitic acid, stearic acid, nonadecanoic acid, arachidic acid, a fatty acid with 21 carbon atoms (C21:0), and behenic acid or unsaturated fatty acids such as oleic acid. Still more preferred amongst these are palmitic acid and stearic acid, each of them because of their particularly excellent botulinum toxin neutralizing abilities and the wide availability on a commercial basis of these acids, which are abundant in animal and vegetable tissues.

Tests have also been conducted on a clinical basis to determine the minimum doses of the botulinum toxin neutralizer according to the present invention as required for the complete neutralization of given quantities of botulinum toxin. These tests have revealed that approximately 50 micrograms of fatty acid with 12 or more carbon atoms is required for the neutralization of 2 micrograms of botulinum toxin. Doses of this order provide a botulinum toxin neutralizing ability approximately equal to one tenth of the antitoxic ability achievable by the botulinum toxin neutralizer of ganglioside GT1b. It is thus considered that a botulinum toxin neutralizer according to the present invention should be used with doses ranging from about 0.5 mg to about 100 mg for the prevention or treatment of botulism.

Fatty acids with 12 or more carbon atoms are generally stable in chemical nature, as is well known in the art, and can therefore be easily manufactured into products ready for use as clinical pharmaceuticals or as additives to foods. Such pharmaceuticals may be provided in the forms of tablets, encapsulated medicines, sugar-coated drugs, powders and granules, syrups, vialed liquids, suppositories, ointments, intravascular injection preparations and so on.

Tests have further been conducted with palmitic acid and ganglioside GT1b by the TLC immunostaining method to evaluate their abilities to combine with the botulinum type A toxin. The results of these tests are demonstrated in the accompanying drawing. The transverse axis indicates quantities of the sample solutions of palmitic acid and ganglioside GT1b in terms of nano mole while the vertical axis represents the integral values of the peaks observed by the densitometric tests conducted on each of the sample solutions. Thus, the plots shown indicate the relative values representative of the toxin neutralizing abilities of both ganglioside GT1b and palmitic acid when 10 micrograms of each of these toxin neutralizing substances is used as a receptor for botulinum toxin.

It may also be noted that tests have further been conducted by the TLC immunostaining method on such substances as cholesterol, cholesterol esters, triglycerides and phospholipids, none of which has however proved to have a botulinum toxin neutralizing ability.

From the foregoing description it will have been understood that the present invention proposes a useful botulinum toxin neutralizer which is easy to manufacture in various forms. The toxin neutralizer acts as if it were an antagonistic receptor for botulinum toxin and, when brought into contact with botulinum toxin in human body, directly combines with the toxin and disables it from combining with the neuromuscular tissues of human body, and thus prevents botulism. Such a botulinum toxin neutralizer can be manufactured economically from a naturally-occurring glyceride, and is for this reason far less costly than the antitoxin of ganglioside produced from bovine brain. The fact that a botulinum toxin neutralizer according to the present invention can be manufactured from a naturally-occurring substance is important because the substance, which is typically a simple fatty acid, usually provides assurance of pathologic safety to human being. Thus a botulinum toxin neutralizer according to the present invention will contribute significantly to the prevention and treatment of botulism.

## Claims

1. The use of fatty acids having at least 12 carbon atoms for the manufacture of a medicament for the treatment of botulism.

2. Use according to claim 1, **characterized in that** the medicament is employed for the prevention of botulism.

3. Use according to claim 1, **characterized in that** the fatty acid possesses one or more of the features:
(A) it is a saturated fatty acid;
(B) it is a straight-chain fatty acid; and
(C) it has 16-22 carbon atoms.

4. Use according to claim 1 or claim 2, **characterized in that** the fatty acid employed is selected from the group consisting of lauric acid, myristic acid, palmitic acid, stearic acid, nonadecanoic acid, arachidic acid and behenic acid.

5. Use according to claim 1 or claim 2, **characterized in that** the fatty acid employed is palmitic acid and/or stearic acid.

6. Use according to any of the preceding claims for the manufacture of a medicament, **characterized in that** besides the fatty acid(s) serving as botulinus toxin neutralizer it also includes a pharmaceutically-acceptable excipient.

7. Use according to any of the preceding claims for the manufacture of a medicament, **characterized in that** it also includes a further botulinus toxin neutralizer.

8. Use according to any of the preceding claims for the manufacture of a medicament, **characterized in that** it takes the form of tablets, capsules, a sugar-coated drug, granules, a syrup, a vialed liquid, suppositories, an ointment or an intravascular injection preparation.

9. Use according to any of the preceding claims for the manufacture of a medicament, **characterized in that** it is in unit dosage form.

10. Use according to claim 8, **characterized in that** each unit dose contains 0.5 to 100 mg of the fatty acid.

11. Use according to any of claims 1 to 7, **characterized in that** the medicament is incorporated in food.

## Patentansprüche

1. Verwendung von Fettsäuren mit mindestens 12 Kohlenstoffatomen zur Herstellung eines Medikaments zur Behandlung von Botulismus.

2. Verwendung nach Anspruch 1, **dadurch gekennzeichnet**, daß das Medikament zur Vorbeugung gegen Botulismus eingesetzt wird.

3. Verwendung nach Anspruch 1, **dadurch gekennzeichnet**, daß die Fettsäure
(A) eine gesättigte Fettsäure ist,
(B) eine unverzweigte Fettsäure ist und/oder
(C) 16-22 Kohlenstoffatome besitzt.

4. Verwendung nach Anspruch 1 oder Anspruch 2, **dadurch gekennzeichnet**, daß die eingesetzte Fettsäure aus der Gruppe bestehend aus Laurinsäure, Myristinsäure, Palmitinsäure, Stearinsäure, Nonadecansäure, Arachinsäure und Behensäure ausgewählt wird.

5. Verwendung nach Anspruch 1 oder Anspruch 2, **dadurch gekennzeichnet**, daß als eingesetzte Fettsäure Palmitinsäure und/oder Stearinsäure verwendet wird.

6. Verwendung nach irgendeinem der vorangehenden Ansprüche zur Herstellung eines Medikamentes, **dadurch gekennzeichnet**, daß es neben der/den als Botulinustoxin-Neutralisator dienenden Fettsäure/Fettsäuren auch einen pharmazeutisch akzeptablen Arzneimittelträger einschließt.

7. Verwendung nach irgendeinem der vorangehenden Ansprüche zur Herstellung eines Medikamentes, **dadurch gekennzeichnet**, daß es noch einen weiteren Botulinustoxin-Neutralisator einschließt.

8. Verwendung nach irgendeinem der vorangehenden Ansprüche zur Herstellung eines Medikamentes, **dadurch gekennzeichnet**, daß es in Form von Tabletten, Kapseln, einem Dragee mit Zuckerüberzug, Granula, einem Sirup, einer Ampullen-Flüssigkeit, Zäpfchen, einer Salbe oder einem intravaskulären Injektionsmittel vorliegt.

9. Verwendung nach irgendeinem der vorangehenden Ansprüche zur Herstellung eines Medikaments, **dadurch gekennzeichnet**, daß es in einer Einheits-Dosierungsform vorliegt.

10. Verwendung nach Anspruch 8, **dadurch gekennzeichnet**, daß jede Einheitsdosis 0,5 bis 100 mg der Fettsäure enthält.

11. Verwendung nach irgendeinem der Ansprüche 1 bis 7, **dadurch gekennzeichnet**, daß das Medikament in einem Nahrungsmittel enthalten ist.

## Revendications

1. Utilisation d'acides gras ayant au moins 12 atomes de carbone pour la fabrication d'un médicament destiné au traitement du botulisme.

2. Utilisation selon la revendication 1, caractérisée en ce que le médicament est employé pour la prévention du botulisme.

3. Utilisation selon la revendication 1, caractérisée en ce que l'acide gras possède une ou plusieurs des caractéristiques suivantes :
(A) c'est un acide gras saturé ;
(B) c'est un acide gras à chaîne linéaire ; et
(C) il a de 16 à 22 atomes de carbone.

4. Utilisation selon la revendication 1 ou la revendication 2, caractérisée en ce que l'acide gras employé est choisi dans l'ensemble constitué par l'acide laurique, l'acide myristique, l'acide palmitique, l'acide stéarique, l'acide nonadécanoïque, l'acide arachidique et l'acide béhénique.

5. Utilisation selon la revendication 1 ou la revendication 2, caractérisée en ce que l'acide gras employé est l'acide palmitique et/ou l'acide stéarique.

6. Utilisation selon l'une quelconque des revendications précédentes pour la fabrication d'un médicament, caractérisée en ce qu'en sus du ou des acides gras servant de neutraliseurs de la toxine botulinique il comprend aussi un excipient pharmaceutiquement acceptable.

7. Utilisation selon l'une quelconque des revendications précédentes pour la fabrication d'un médicament, caractérisée en ce qu'il comprend aussi un neutraliseur de la toxine botulinique supplémentaire.

8. Utilisation selon l'une quelconque des revendications précédentes pour la fabrication d'un médicament, caractérisée en ce que celui-ci prend la forme de comprimés, de capsules, d'un médicament enrobé de sucre, de granulés, d'un sirop, d'un liquide en ampoule, de suppositoires, d'une pommade ou d'une préparation pour injection intravasculaire.

9. Utilisation selon l'une quelconque des revendications précédentes pour la fabrication d'un médicament, caractérisée en ce que celui-ci est sous la forme d'unité de prise.

10. Utilisation selon la revendication 8, caractérisée en ce que chaque unité de prise contient de 0,5 à 100 mg de l'acide gras.

11. Utilisation selon l'une quelconque des revendications 1 à 7, caractérisée en ce que le médicament est incorporé à la nourriture.
